# EUROPEAN PATENT SPECIFICATION

(11) **EP 0 939 624 B1**
(45) Date of publication and mention of the grant of the patent: **17.12.2003**
(21) Application number: 97924695.6
(22) Date of filing: 13.05.1997
(51) Int. Cl.: A61K 9/46, A61K 31/66

(54) **EFFERVESCENT BISPHOSPHONATE FORMULATION**
BIPHOSPHONAT-FORMULIERUNG MIT BRAUSEWIRKUNG
FORMULATION DE BISPHOSPHONATE EFFERVERSCENTE

(30) Priority: 17.05.1996 US 17881 P; 24.06.1996 GB 9613183
(43) Date of publication of application: 08.09.1999
(60) Divisional application: 03077124.0
(73) Proprietor: MERCK & CO., INC., Rahway, New Jersey 07065 (US)
(72) Inventor: KATDARE, Ashok, V., Rahway, NJ 07065 (US); KRAMER, Kenneth, A., Rahway, NJ 07065 (US); GARDNER, Colin, R., Rahway, NJ 07065 (US)
(74) Representative: Hiscock, Ian James
(86) International application number: US9708055
(87) International publication number: WO97044017

(56) References cited:
- EP-A- 0 521 388
- WO-A-93/11774
- WO-A-94/14455
- WO-A-96/41618
- GB-A- 2 153 225
- US-A- 4 621 077
- US-A- 5 270 365

## Description

### FIELD OF THE INVENTION

This invention relates to pharmaceutical effervescent formulations of alendronate, and in particular, tablets which effervesce when added to water.

### BACKGROUND OF THE INVENTION

There are numerous bisphosphonates which have been identifed as having utility as pharmaceutical agents which inhibit bone resorption. These include:
alendronate--(4-amino-1-hydroxy-butylidene)bis-phosphonate;
cimadronate--[(cycloheptylamino)methylene]bis-phosphonate;
clodronate--(dichloromethylene)-bis-phosphonate;
EB-1053--[1-hydroxy-3-(1-pyrrolidinyl)-propylidene]bis-phosphonate;
etidronate--(1-hydroxyethylidene)-bis-phosphonate;
ibandronate--[1-hydroxy-3-(methylpentylamino)propylidene]bis-phosphonate;
neridronate--(6-amino-1-hydroxyhexylidene)bis-phosphonate;
olpadronate--[3-(dimethylamino)-1-hydroxy-propylidene]bis-phosphonate;
pamidronate--(3-amino-1-hydroxypropylidene)bis-phosphonate;
risedronate--[1-hydroxy-2-(3-pyridinyl)-ethylidene]bis-phosphonate;
tiludronate--[[(4-chlorophenyl)thio]methylene]bis-phosphonate
YH 529--[1-hydroxy-2-imidazo-(1,2a)pyridin-3-ylethylidene]bis-phosphonate; and
zoledronate--[1-hydroxy-2-(1H-imidazol-1-yl)ethylidene]bis-phosphonate.

Alendronate has been approved by various regulatory agencies, including the Food and Drug Administration in the United States as an oral osteoporosis treatment in post menopausal women. The currently marketed formulation is a tablet, and the patient is instructed to take the tablet with a full glass of water in the morning, at least a half hour prior to eating or drinking. However, certain side effects, including esophageal irritation and erosion have been reported if the tablet was not taken with enough water, or if the patient did not remain in an upright position for approximately one-half hour after taking the medication.

It would be desirable to develop a formulation which allows a bisphosphonate to be taken orally, yet avoids problems associated with prolonged contact between the medication and the esophagus.

U.S. Patent No. 4,621,077 discloses pharmacologically active bisphosphonates, process for the preparations thereof and pharmaceutical compositions therefrom. U.S. Patent No. 5,270,365 teaches the prevention and treatment of periodontal disease with alendronate.

### DETAILED DESCRIPTION OF THE INVENTION

The present invention is directed to an effervescent tablet formulation comprising (in mg),

| | |
|---|---|
| Alendronate sodium (in mg alendronic acid) | 2.5-10 |
| citric acid | 400-800 |
| sodium bicarbonate | 1000-2000 |
| sodium carbonate | 20-80 |
| sodium benzoate | 25-175 |
| xylitol | 100-300. |

As used throughout this specification and claims, the term "Alendronate" includes the related bisphosphonic acid, and salt forms. It includes crystalline, hydrated crystalline, and amorphous forms of alendronate. It specifically includes alendronate sodium and alendronate monosodium trihydrate.

Methods for the preparation of alendronate and alendronate sodium salt trihydrate are known. In particular, methods for the preparation of alendronate may be found in U.S. patents 4,922,007, 5,019,651 and 5,510,517. Exemplary amounts of alendronate are 5 and 10 mg.

The formulations of this invention have many distinct advantages. First of all, the patients drink an effervescent liquid, which limits the amount of time in which the bisphosphonate is in contact with the esophageal tissue, thus minimizing the risk of irritation. Secondly, the bioavailability of alendronate is increased as a consequence of an excipient acting as a sequestering agent. Thirdly, the alendronate formulations are particularly advantageous in that alendronate is often prescribed to elderly patients who may experience difficulty in swallowing pills, but can more easily swallow a liquid formulation.

In preferred feature of the present invention, citric acid is employed as an acid source which is also a sequestering agent. This is an important consideration because alendronate can be a potent sequestering agent of divalent cations, especially Ca²⁺ and Mg²⁺. If either of these cations are present, the alendronate will sequester them, rendering the alendronate less bioavailable. The excess citric acid in the formulation binds the ions in issue and prevents their complexation with alendronate.

A carbonate source is chosen so that it does not contain divalent cations which could be sequestered by the alendronate. The chosen carbonate sources are sodium bicarbonate, sodium carbonate, and mixtures thereof.

In one aspect of this invention the acid source is present in an amount equal or greater than the carbonate source, on a molecular equivalent basis. Thus, when citric acid is the acid source and sodium bicarbonate is the carbonate source, the mole ratio of citric acid/bicarbonate is at least 1:3. In a particular class of this aspect there is an excess of citric acid, as this not only helps to efficiently generate the effervescence, but also acts to sequester any ions which might otherwise complex with alendronate and the excess also acts as a flavor enhancer. In an illustration of the excess acid source, the mole ratio of citric acid to bicarbonate ranges from 1: to 3:1. Where sodium carbonate is used as the source of carbonate an equal equivalent with citric acid will require a mole ratio of 2 moles citric acid to 3 moles carbonate. Analogous ratios can be calculated for any source of acid and carbonate, and of course the carbonate source may be present as a mixture of bicarbonate and carbonate.

For patients who exhibit gastric irritation another aspect of this invention is to employ excess amounts of the carbonate source to provide an antacid effect to the formulation.

A binder, sodium benzoate, is also present. The binder is usually present in approximately 3-10% of the tablet, and preferably about 5% (based on wt/wt), e.g. if the tablet weighs 1000 mg, the binder will be from about 30-100 mg, more preferably about 50 mg.

The tablet should also contain a lubricant, which typically comprises about 0.5 to about 5% of the tablet on a wt/wt basis, and preferably about 1%. The sodium benzoate also functions as a lubricant.

Where desired a colorant, flavoring agent such as orange or cherry or sweetener (in addition to xylitol), such as aspartame, or acesulfame K, may be added to the formulation. Various methodologies for preparing the formulations of the current invention are illustrated in the examples below.

The following formulation and manufacturing procedure can be used for manufacture of effervescent tablets containing alendronate sodium.

### EXAMPLE 1

| | | |
|---|---|---|
| Alendronate sodium (in mg alendronate acid) | 2.5-10 | 10 mg |
| Citric acid, anhydrous (granular) | 400-800 | 600 mg |
| Sodium bicarbonate (granular) | 1000-2000 | 1500 mg |
| Sodium carbonate, anhydrous | 20-80 | 40 mg |
| Flavoring agent (optional) | 0-50 | 25 mg |
| Colorant (optional) | 0-15 | 5 mg |
| Sodium benzoate | 25-175 | 150 mg |
| Xylitol | 100-300 | 200 mg |
| Sweetener (aspartame) | 1-10 | 2.5 mg |

Part 1. Blend in a suitable mixer xylitol, alendronate and 400 mg sodium bicarbonate. Roller compact this mixture.

Part 2. Blend in a suitable mixer sodium benzoate, citric acid, 900 mg sodium bicarbonate, color, flavor and sweetener; then roller compact this mixture.

Part 3. Granulate Parts 1 and 2 through a suitable screen, add remaining powders and mix.
Compress the granulation using a suitable tooling. Package the tablets in aluminum foil.

## Claims

1. A formulation comprising (in mg):
| | |
|---|---|
| Alendronate sodium (in mg alendronic acid) | 2.5-10 |
| citric acid | 400-800 |
| sodium bicarbonate | 1000-2000 |
| sodium carbonate | 20-80 |
| sodium benzoate | 25-175 |
| xylitol | 100-300. |

2. A formulation according to Claim 1 further comprising one or more flavoring agents, colorants and/or additional sweeteners.

## Patentansprüche

1. Eine Formulierung, umfassend (in mg):
| | |
|---|---|
| Alendronat-Natrium (in mg Alendronsäure) | 2,5 - 10 |
| Zitronensäure | 400 - 800 |
| Natriumhydrogencarbonat | 1000 - 2000 |
| Natriumcarbonat | 20 - 80 |
| Natriumbenzoat | 25 - 175 |
| Xylit | 100 - 300. |

2. Eine Formulierung gemäß Anspruch 1, ferner umfassend einen oder mehrere Geschmacksstoffe, Farbstoffe und/oder zusätzliche Süßstoffe.

## Revendications

1. Formulation comprenant (en mg) :
| | |
|---|---|
| Alendronate de sodium (en mg d'acide alendronique) | 2,5 à 10 |
| Acide citrique | 400 à 800 |
| Bicarbonate de sodium | 1000 à 2000 |
| Carbonate de sodium | 20 à 80 |
| Benzoate de sodium | 25 à 175 |
| Xylitol | 100 à 300 |

2. Formulation selon la revendication 1, comprenant en outre un ou plusieurs agents parfumant, colorants et/ou édulcorants supplémentaires.
